# EUROPÄISCHE PATENTSCHRIFT

(11) **EP 2 261 649 B1**
(45) Veröffentlichungstag und Bekanntmachung des Hinweises auf die Patenterteilung: **26.12.2018**
(21) Anmeldenummer: 10004546.7
(22) Anmeldetag: 29.04.2010
(51) Int. Cl.: G01N 27/416

(54) **Verfahren und Schaltung zur Prüfung eines Kohlenmonoxid-Sensors**
Method and circuit for testing a carbon monoxide sensor
Procédé et circuit pour la vérification d'un capteur de monoxyde de carbone

(30) Priorität: 10.06.2009 DE 102009024573
(43) Veröffentlichungstag der Anmeldung: 15.12.2010
(73) Patentinhaber: Novar GmbH, 41469 Neuss (DE)
(72) Erfinder: Politze, Heiner, 41469 Neuss (DE); Ueding, Markus, 41749 Viersen (DE)
(74) Vertreter: Henkel, Breuer & Partner

(56) Entgegenhaltungen:
- EP-A1- 1 039 293
- EP-A2- 0 039 549
- WO-A1-90/12315
- WO-A1-03/001195

## Beschreibung

Die Erfindung betrifft ein Verfahren der im Oberbegriff des Anspruches 1 angegebenen Art und eine Schaltung gemäß dem Oberbegriff des Anspruches 5.

Ein häufig zur Ermittlung der Kohlenmonoxidkonzentration in Luft oder einer Gasatmosphäre verwendeter, auf elektrochemischer Basis arbeitender Sensor umfasst im wesentlichen zwei, teilweise auch drei Elektroden, einen mit einem sauren Elektrolyten getränkten Schwamm und einen Kohlefilter in einem kleinen Gehäuse mit einer Öffnung, über die der Luft- bzw. Gasaustausch mit der Umgebung erfolgt. Die Elektroden sind mit einer Schaltung verbunden, die üblicherweise einen Differenzverstärker umfasst, dessen Ausgangssignal ein Maß für die Kohlenmonoxidkonzentration ist.

In der Regel ist ein derartiger Kohlenmonoxid-Sensor, im Folgenden kurz "Sensor", integraler Bestandteil eines Gaswarngeräts und insbesondere eines Brandmelders, der auch auf andere Brandkenngrößen als die Entwicklung von Kohlenmonoxid anspricht. Der Sensor sitzt gewöhnlich gesockelt auf einer Schaltungsplatine. Die ordnungsgemäße Funktion des betreffenden Gerätes ist nicht gewährleistet, wenn
- der Sensor fehlt, z.B. sich als Folge starker Erschütterungen aus seinem Sockel gelöst hat,
- der Sensor z.B. wegen Lötfehlers kurzgeschlossen ist,
- der Elektrolyt in extrem trockener Umgebung oder nach sehr langer Betriebszeit mindestens zum Teil ausgetrocknet ist
- oder umgekehrt der Elektrolyt in sehr feuchter Umgebung durch Wasseraufnahme verdünnt ist.

Aus der DE 690 23 129 T2 (= EP 467 902 B1 = US 5 202 637 A) sind ein verfahren und eine Schaltung zur Funktionsprüfung eines elektrochemischen Sensors mit drei Elektroden, nämlich einer Arbeitselektrode, einer Gegenelektrode und einer Referenzelektrode, bekannt. Zur Funktionsprüfung wird die Offset-Spannung des nachgeschalteten Differenzverstärkers, dessen Ausgang mit der Gegenelektrode verbunden ist, für einen Bruchteil einer Millisekunde auf einen Wert umgeschaltet, bei dem zwischen der Referenzelektrode und der Abtastelektrode ein Potentialunterschied erzeugt wird, der bei intaktem Sensor zu einem kapazitiven umladevorgang in dem Sensor führt. Der dadurch entstehende Strom bedeutet, dass der Sensor funktionsfähig ist.

Aus der EP 039 549, Fig. 2 sind ein verfahren und eine Schaltung der einleitend angegebenen Gattung bekannt. Der Elektrolyt des Sensors besteht aus einer wässrigen Lösung eines Jod- oder Bromsalzes, zur Funktionsprüfung werden die Sensorelektroden für einige Millisekunden auf eine Prüfspannungsquelle umgeschaltet. Die angelegte Spannung erzeugt durch elektrolytische Zerlegung Salzionen, die nach dem Abschalten der prüfspannung rekombinieren, wodurch der Sensor einen erhöhten Ausgangsstrom abgibt. Prüfkriterium ist, ob sich der Ausgangsstrom des Differenzverstärkers entsprechend dem Prüfimpuls ändert. Das Verfahren und die Schaltung erfordern jedoch eine zum Bezugspotential symmetrische Versorgungsspannung von ± 15 v und vor allem eine zweipolige Umschaltung des Sensors.

Aus der EP 1 039 293 A1 ist ein verfahren mit den Merkmalen des Oberbegriffes des Anspruches 1 bekannt. Es beruht darauf, dass bei einem intakten CO-Sensor das im normalen Betrieb gemessene Ausgangssignal sich zu dem im inversen Betrieb des Sensors erzeugten Ausgangssignal wie 1 : 1 verhält. Durch geeignete wahl der Dauer der Prüfspannung und einer Wartezeit nach dem Abschalten der prüfspannung kann aus dem dann gemessenen Ausgangssignal ein Korrekturwert für die CO-Konzetitration ermittelt werden.

Der Erfindung liegt die Aufgabe zugrunde, ein genaueres Verfahren und eine vereinfachte schaltung zur Funktionsprüfung des Sensors zur Verfügung zu stellen.

Verfahrensmäßig ist diese Aufgabe dadurch gelöst, dass das Ausgangssignal zu einem vorgegebenen Zeitpunkt nach dem Abschalten der Prüfspannung gemessen, der Messwert mit einem als Mindestwert vorgegebenen Bezugswert verglichen und bei Unterschreitung dieses Mindestwertes ein Fehlersignal erzeugt wird.

Das Verfahren macht sich die Tatsache zunutze, dass der Strom, den der Sensor nach dem Abschalten der Prüfspannung analog einer Brennstoffzelle in einen äußeren Stromkreis liefert, näherungsweise entsprechend einer e-Funktion abklingt. Wegen dieses zeitlichen Verlaufes muss folglich bei einem intakten Sensor der Messwert des Ausgangssignals zu einem gegebenen Zeitpunkt größer als ein zuvor empirisch ermittelter Mindestwert sein. Wird der Mindestwert unterschritten, liegt einer der drei folgenden Fehler vor:
(1) der Sensor fehlt
(2) der Sensor ist innerlich oder äußerlich kurzgeschlossen
(3) der Elektrolyt ist über die Grenze der Funktionsfähigkeit hinaus ausgetrocknet.

Zusätzlich kann das Ausgangssignal zu mindestens einem vorgegebenen Zeitpunkt nach dem Anlegen der Prüfspannung gemessen, der Messwert mit einem vorgegebenen oberen Bezugswert verglichen und bei Unterschreitung des Bezugswertes ein Fehlersignal, zweckmäßig ein für diese Messung signifikantes drittes Fehlersignal, erzeugt werden. Der für diesen Vergleich vorgegebene Bezugswert kann höher als der oben genannte Bezugswert sein, mit dem der Messwert zu dem vorgegebenen Zeitpunkt nach dem Abschalten der Prüfspannung verglichen wird. Durch diese weitere Messung wird nicht der Sensor selbst geprüft sondern es wird ermittelt, ob die Prüfspannung vorhanden ist und der Differenzverstärker ordnungsgemäß arbeitet. Die gleiche Messung kann kurze Zeit nach dem Abschalten der Prüfspannung wiederholt werden. Wenn der Messwert dann kleiner als der (gleiche) Bezugswert ist, bedeutet dies, dass der Sensor nicht ausreichend polarisiert wurde, was ebenfalls auf einen zu trockenen Elektrolyt hinweist.

Bevorzugt wird das Ausgangssignal auch vor dem Anlegen der Prüfspannung gemessen und der Messwert mit einem Mindestwert oder unteren Grenzwert verglichen sowie bei Überschreitung dieses unteren Grenzwertes ein Fehlersignal, vorzugsweise ein von den anderen Fehlersignalen verschiedenes Fehlersignal, erzeugt. Wenn dieser Messwert den unteren Grenzwert, der der gleiche wie der Bezugswert im Fall der ersten oben genannten Messung sein kann, überschreitet, bedeutet dies, dass der Sensor bereits im normalen Betrieb ein über dem Ruhesignal liegendes Signal erzeugt. Die wahrscheinlichste Ursache ist, dass der Sensor bereits auf eine erhöhte Kohlenmonoxidkonzentration, verursacht z.B. durch einen beginnenden Brand, reagiert.

Schaltungsmäßig ist die eingangs genannte Aufgabe dadurch gelöst, dass die eine (erste) Elektrode des Sensors über eine Klemmdiode mit dem Bezugspotential (Masse) der Schaltung verbunden ist, das die andere (zweite) Elektrode im normalen Betriebszustand auf einem gegenüber dem Bezugspotential positiven Potential zwischen 0,1 V und 1 V liegt und zur Funktionsprüfung auf ein dem gegenüber höheres positives Potential der Prüfspannungsquelle umschaltbar ist, und dass der Ausgang des Differenzverstärkers mit dem Eingang eines Vergleichers verbunden ist.

Die Klemmdiode, z.B. eine LED, hält die erste Elektrode des Sensors auf einem festen positiven Potential von z.B. 1,6 V gegenüber dem Bezugspotential. Die zweite Elektrode, die im normalen Messbetrieb des Sensors auf einem kleinen Potential von in der Regel einigen 100 Millivolt gehalten wird und damit gegenüber der ersten Elektrode negativ ist, kann folglich auf ein höheres positives Potential als die erste Elektrode gebracht werden, das die Prüfspannungsquelle liefert und das bei der häufig verwendeten Betriebsspannung von 3,3 V nahe dieser Betriebsspannung liegen kann. Die Spannungsdifferenz zu dem Potential der ersten Elektrode ist völlig ausreichend für die elektrolytische Zerlegung des Elektrolyt des Sensors.

Die Schaltung nach der Erfindung erfordert daher keine von üblichen CMOS-Schaltungen abweichende Betriebsspannung und verwirklicht mit einfachen Mitteln die für die Funktionsprüfung notwendige Umschaltung der Potentiale an den Elektroden des Sensors.

Konkret kann die andere (zweite) Elektrode des Sensors mit dem Abgriff eines Widerstandsspannungsteilers verbunden sein, dessen einer Anschluss mit einem Anschluss der Prüfspannungsquelle verbunden ist, der im Betrieb auf dem Bezugspotential (Masse) und zur Prüfung auf einem positiven Potential z.B. nahe der Betriebsspannung der Schaltung liegt, wobei an dem anderen Anschluss des Widerstandsspannungsteilers die Betriebsspannung anliegt. Kurz gesagt, werden also die unterschiedlichen Potentiale der zweiten Elektrode des Sensors im Messbetrieb und während der Funktionsprüfung durch einen zwischen dem Bezugspotential und einem positiven Pegel umschaltbaren Anschluss der Prüfspannungsquelle erzielt.

Bei einer bevorzugten Ausführungsform der Schaltung ist der Ausgang des Differenzverstärkers mit dem Eingang eines A/D-Wandlers eines Mikroprozessors verbunden, der einen zwischen dem Bezugspotential und einem positiven Potential der Prüfspannungsquelle umschaltbaren Ausgang hat sowie einen Speicher für einen den Ablauf und die Auswertung der Funktionsprüfung steuerndes Programm umfasst.

In dieser Ausführungsform der Schaltung eignet sich der Sensor insbesondere zur Integration in einen Brandmelder oder ein Gaswarngerät, dessen übrige Funktionen von dem gleichen Mikroprozessor gesteuert und überwacht werden.

Das Verfahren und die Schaltung nach der Erfindung werden nachfolgend anhand der Zeichnung erläutert. Es zeigt:
Fig. 1: ein Ausführungsbeispiel der Schaltung und
Fig. 2: zwei Spannungs/Zeit-Diagramme zur Erläuterung des Funktionsprüfverfahrens.

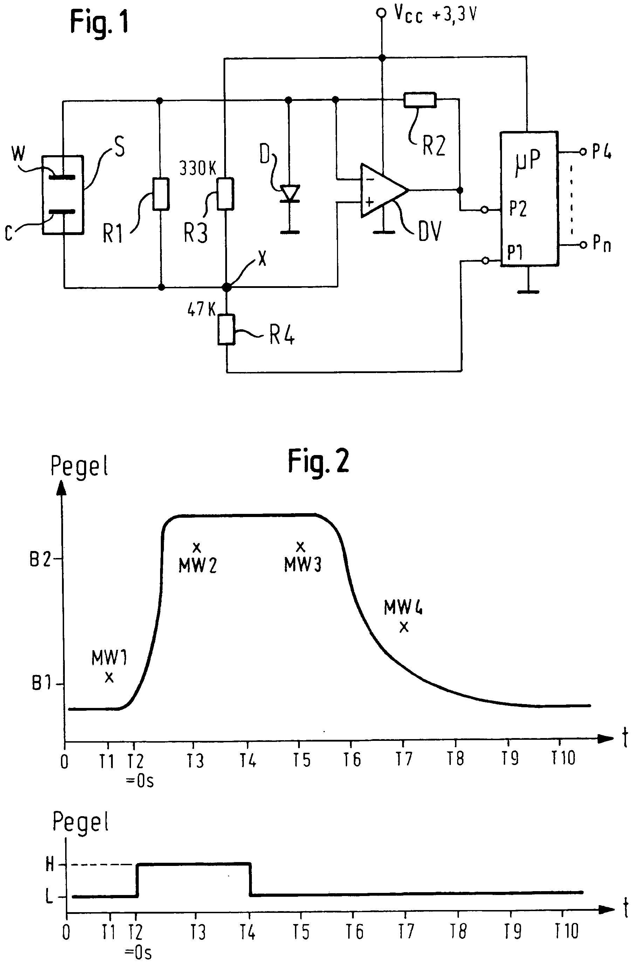

Die Schaltung gemäß Figur 1 kann insbesondere Bestandteil eines Brandmelders sein, der neben der Erkennung von anderen Brandkenngrößen auf die Entstehung von Kohlenmonoxid anspricht. Die Schaltung umfasst einen handelsüblichen Kohlenmonoxid-Sensor S mit einer Arbeitselektrode W und einer Gegenelektrode C. Die Arbeitselektrode W ist im Normalbetrieb auf einem Potential, das positiver als dasjenige der Gegenelektrode C ist. Im Normalbetrieb ist der Sensor S niederohmig, wirkt also näherungsweise wie eine Stromquelle. Die Elektroden sind mit einem Widerstand R1 von überbrückt, der eine Polarisierung der Sensorelektroden in Betriebspausen verhindert. Die Gegenelektrode C ist mit dem positiven Eingang eines Differenzverstärkers DV, z.B. eines handelsüblichen Operationsverstärkers, verbunden, die Arbeitselektrode W mit dessen negativen Eingang. Der Ausgang des Differenzverstärkers DV ist wie üblich über einen Gegenkopplungswiderstand R2 mit dem negativen Eingang verbunden.

Die Gegenelektrode C des Sensors S und damit auch der positive Eingang des Differenzverstärkers DV sind mit dem Abgriff X eines Spannungsteilers aus den Widerständen R3 und R4 verbunden. Der andere Anschluss des Widerstandes R3 ist mit der positiven Betriebsspannung Vcc von z.B. 3,3 V verbunden. Der andere Anschluss des Widerstandes R4 ist mit einem Port 1 eines Mikroprozessors *µ*P verbunden. Der Mikroprozessor arbeitet mit der Betriebsspannung Vcc. Der Port 1 liegt im normalen Betrieb auf L (LOW) nahe dem Bezugspotential (Masse) und ist, wie noch erläutert werden wird, zur Funktionsprüfung auf H (HIGH) umschaltbar. Der Pegel H liegt wie üblich nahe bei Vcc.

Der Spannungsteiler R3, R4 ist so dimensioniert, dass die Gegenelektrode C des Sensors S auf einem kleinen positiven Potential, z.B. 0,4 V liegt. Zur Funktionsprüfung, d.h. wenn der Port 1 des Mikroprozessors auf H umgeschaltet ist, liegt die Gegenelektrode stattdessen nahezu auf Vcc.

Die Arbeitselektrode W und damit der negative Eingang des Differenzverstärkers DV sind über eine LED D mit dem Bezugspotential verbunden. Dadurch wird die Arbeitselektrode W auf ein Potential entsprechend der Diodendurchlassspannung, also je nach Diodentyp auf etwa 1,6 V geklemmt, d.h. hier auf etwa die Hälfte der Betriebsspannung. Folglich wird zur Funktionsprüfung das Potential an der Gegenelektrode C in Bezug auf die Arbeitselektrode W invertiert und liegt um ca. 1,6 V höher als dieses Potential.

Der Ausgang des Differenzverstärkers DV ist mit einem Port 2 des Mikroprozessors verbunden, der weitere Ports 3 bis n hat, die z.B. im Fall eines Brandmelders weitere Mess-, Steuerungs- und Kommunikationsfunktionen erfüllen. Das analoge Ausgangssignal des Differenzverstärkers DV verarbeitet den Mikroprozessor *µ*P nach Digitalisierung entsprechend dem anhand von Figur 2 erläuterten Ablaufdiagramm weiter.

Im normalen Messbetrieb ist das Ausgangssignal des Differenzverstärkers DV im Wesentlichen proportional zu der Kohlenmonoxidkonzentration, der der Sensor S ausgesetzt ist.

Zur Funktionsprüfung wird der Port 1 des Mikroprozessors *µ*P auf H geschaltet, damit auch der positive Eingang des Differenzverstärkers DV und die Gegenelektrode C. Das am Port 2 anliegende Ausgangssignal des Differenzverstärkers DV muss deshalb ebenfalls H sein. Dies prüft der Mikroprozessor.

Solange die Gegenelektrode C des Sensors S auf H ist, findet in dem Sensor S eine Elektrolyse statt. Nach dem Umschalten des Ports 1 auf L bleibt die Gegenelektrode C des nun wie eine Brandstoffzelle arbeitenden Sensors S noch auf dem positiven Potential, das jedoch sehr bald nach einer e-Funktion abklingt und proportional dazu auch das Ausgangssignal des Differenzverstärkers DV.

Der Verfahrens- und Programmablauf während der Funktionsprüfung ist in Figur 2 schematisch dargestellt.

Im oberen und im unteren Diagramm ist auf der Abszisse die Zeit t aufgetragen.

Im oberen Diagramm ist auf der Ordinate das Ausgangssignal des Differenzverstärkers DV aufgetragen, das am Port 2 des Mikroprozessors *µ*P anliegt und von diesem ausgewertet wird. Im unteren Diagramm ist auf der Ordinate der Pegel L bzw. H am Port 1 des Mikroprozessors *µ*P aufgetragen. Die Punkte MW1 bis MW4 bezeichnen keine quantitativen Messwerte sondern stehen symbolisch für zu den betreffenden Zeitpunkten erfolgende Messungen der jeweiligen Signalpegel.

Die Funktionsprüfung des Sensors kann als Einschaltkontrolle, d.h. bei der ersten oder einer erneuten Inbetriebnahme des Sensors oder eines damit ausgerüsteten Gerätes, wahlweise auch in periodischen Zeitabständen, selbsttätig durchgeführt werden. Fallweise kann auf einzelne der nachfolgend beschriebenen Mess- bzw. Prüfschritte auch verzichtet werden.

Zur Durchführung einer Funktionsprüfung wird unmittelbar vor deren Beginn, zu einem Zeitpunkt T1, der Pegel des Ausgangssignals des Differenzverstärkers DV als erster Messwert MW 1 bestimmt und durch Vergleich ermittelt ob dieser Messwert MW 1 unter einem vorgegebenen unteren Bezugswert B1 liegt, der höher als der größte zu erwartende Ruhewert ist. Der Bezugswert B1 berücksichtigt, dass der Ruhewert von Exemplarstreuungen des Sensors, der zulässigen ("normalen") Kohlenmonoxidkonzentration, der Temperatur und anderen äußeren Parametern beeinflusst wird. Wenn MW 1 größer als der Bezugswert B1 ist, liegt entweder schon eine zu hohe CO-Konzentration oder eine Funktionsstörung vor. In beiden Fällen hat eine Durchführung der Funktionskontrolle keinen Sinn. Stattdessen wird ein erstes Fehlersignal erzeugt, dass z.B. einen für diesen Zustand spezifischen Alarm an dem Gerät selbst (z.B. dem Brandmelder) und/oder einer Zentrale, mit der das Gerät kommuniziert, auslöst.

Wenn MW 1 kleiner als der Bezugswert B1 ist, wird unmittelbar anschließend zum Zeitpunkt T2 der Port 1 des Mikroprozessors *µ*P gemäß dem unteren Diagramm von L auf H umgeschaltet. Infolgedessen steigt das Signal am Port 2 entsprechend dem oberen Diagramm steil bis auf einen Sättigungswert an. Zum Zeitpunkt T3 wird der Messwert MW 2 genommen und mit einem vorgegebenen oberen Bezugswert B2 verglichen, der kleiner als der Sättigungswert ist. Wenn MW 2 kleiner als der obere Bezugswert B2 ist, können entweder der Sensor oder/und die Schaltung einen Defekt haben. Auch in diesem Fall hat die Fortsetzung der Funktionsprüfung keinen Sinn. Stattdessen wird ein Fehlersignal, das von dem ersten Fehlersignal verschieden sein und dementsprechend einen anderen Alarm auslösen kann, erzeugt.

Zum Zeitpunkt T4 setzt der Mikroprozessor *µ*P den Port 1 zurück auf L. Das Zeitintervall zwischen T2 und T4 ist so gewählt, dass in dieser Zeit eine Elektrolyse in dem Sensor stattgefunden hat, die ausreicht, um ab T4 durch Rekombination eine Spannung an den Elektroden C und W des Sensors S zu erzeugen, die die weitere Funktionsprüfung ermöglicht. Z.B. kann das Zeitintervall zwischen T2 und T4 bei einer Sekunde liegen.

Kurz nach T4, zum Zeitpunkt T5, wird ein Messwert MW3 genommen und mit dem gleichen oberen Bezugswert B2 verglichen. Wenn MW3 kleiner als B2 ist, hat entweder keine ausreichende Elektrolyse in dem Sensor S stattgefunden oder, wenn MW3 gleich 0 ist oder zumindest erheblich unter dem oberen Bezugswert B2 liegt, der Sensor fehlt oder ist kurzgeschlossen. In jedem Fall wird vorzugsweise das gleiche Fehlersignal wie in der entsprechenden Situation zum Zeitpunkt T3 erzeugt.

Ungefähr zum Zeitpunkt T6 ist die Entladung des Sensors soweit fortgeschritten, dass die Spannung zwischen seinen Elektroden C und W und damit die Spannung am Port 2 des Mikroprozessors *µ*P nach einer e-Funktion abzufallen beginnt, wie näherungsweise in dem oberen Diagramm angedeutet. Etwa zum Zeitpunkt T7 wird ein weiterer Messwert MW 4 genommen und mit einem unteren Bezugswert verglichen, der gleich dem oder größer als der für den Vergleich im Zeitpunkt T1 benutzte Bezugswert B1 sein kann. Wenn MW 4 kleiner als dieser Bezugswert ist, hat entweder der Spannungsabfall des Sensors zwar nach T5 aber vor T6 begonnen oder ist zu steil. In beiden Fällen war die Elektrolyse in dem Sensor S nicht ausreichend. Das deutet darauf hin, dass der Elektrolyt zu stark ausgetrocknet ist. Jedenfalls ist der Sensor bei diesem Ergebnis nicht ordnungsgemäß funktionsfähig. Dementsprechend wird wiederum ein Fehlersignal erzeugt, das entweder für dieses Ergebnis spezifisch oder das gleiche wie in den vorhergehenden möglichen Fehlerfällen sein kann.

An T7 schließt sich ein weiteres Zeitintervall bis T10 an, das so bemessen ist, dass ein ordnungsgemäß arbeitender Sensor spätestens ab T10 wieder seinen Ruhewert erreicht haben muss. Der Mikroprozessor *µ*P schaltet folglich ab T10 in den normalen Messbetrieb um.

Die vorstehende, vollständige Funktionsprüfung von T2 bis T10 kann insgesamt bis zu etwa 10 Sekunden dauern. Die Zeitpunkte T2 bis T10, die hier der Einfachheit halber in gleichen Abständen gewählt sind, werden aus dem internen Taktgenerator des Mikroprozessors *µ*P abgeleitet. Die benötigten Bezugswerte sind herstellerseitig in dem Mikroprozessor abgelegt, der auch alle Messwerte und alle Vergleichsergebnisse abrufbar speichert.

Eine vereinfachte Funktionsprüfung kann sich auf die Ermittlung der Messwerte MW1 und MW4 beschränken.

## Patentansprüche

1. Verfahren zur Funktionsprüfung eines elektrochemischen, einen Elektrolyt enthaltenden Gassensors (S), insbesondere eines Kohlenmonoxid-Sensors, dessen Elektroden (C, W) mit einem Differenzverstärker (DV) verbunden sind, dessen Ausgangssignal ein Maß für eine Gaskonzentration ist, wobei zur Funktionsprüfung eine Prüfspannung, die, bezogen auf den normalen Betriebszustand des Sensors invertiert und größer als die Elektrolysespannung des Elektrolyt ist, an die Elektroden angelegt und zumindest nach dem Abschalten der Prüfspannung das Ausgangssignal des Differenzverstärkers gemessen wird, wobei nach dem Abschalten der Prüfspannung das Ausgangssignal zu einem vorgegebenen Zeitpunkt (T7) während des Abklingens des von dem Sensor(S) gelieferten Stroms gemessen, der Messwert (MW4) mit einem vorgegebenen Mindestwert (B1) verglichen und bei Unterschreitung dieses Mindestwerts ein Fehlersignal erzeugt wird.

2. Verfahren nach Anspruch 1, wobei das Ausgangssignal zu mindestens einem vorgegebenen Zeitpunkt (T3, T5) nach dem Anlegen der Prüfspannung und vor der Entladung des Sensors gemessen und der weitere Messwert (MW2, MW3) mit einem vorgegebenen oberen Bezugswert (B2) verglichen wird und wobei bei Unterschreitung dieses oberen Bezugswertes (B2) ein weiteres Fehlersignal erzeugt wird.

3. Verfahren nach Anspruch 1 oder 2, wobei das Ausgangssignal vor dem Anlegen der Prüfspannung gemessen und dieser dritte Messwert (MW1) mit dem vorgegebenen Mindestwert (B1) verglichen wird und wobei bei Überschreitung dieses Mindestwertes (B1) ein drittes Fehlersignal erzeugt wird.

4. Sensorsystem, ausgestaltet zur Durchführung eines Verfahrens zur Funktionsprüfung eines elektrochemischen, einen Elektrolyt enthaltenden Gassensors (S) nach einem der Ansprüche 1 bis 3, wobei das Sensorsystem umfasst:
einen elektrochemischen, einen Elektrolyt enthaltenden Gassensor (S), insbesondere Kohlenmonoxid-Sensor;
einen Differenzverstärker (DV);
eine Klemmdiode (D); und
einen Mikroprozessor (µP) mit einem Vergleicher; wobei
Elektroden (C, W) des Gassensors (S) einerseits mit den Eingängen des Differenzverstärkers (DV) verbunden sind und andererseits zur Funktionsüberprüfung mit einer Prüfspannungsquelle verbunden werden können, die eine Prüfspannung (H) liefert, die, bezogen auf den normalen Betriebszustand des Sensors (S), invertiert und höher als die Elektrolysespannung des Elektrolyt ist; eine erste Elektrode (W) über die Klemmdiode (D) mit dem Bezugspotential (Masse) verbunden ist, eine zweite Elektrode (C) im normalen Betriebszustand auf einem gegenüber dem Bezugspotential positiven Potential zwischen 0,1 V und 1 V liegt und zur Funktionsprüfung auf ein demgegenüber höheres positives Potential der Prüfspannungsquelle umgeschaltet werden kann, da die zweite Elektrode (C) des Gassensors (S) mit dem Abgriff (X) eines Widerstandsspannungsteilers (R3, R4) verbunden ist, dessen einer Anschluss mit einem Anschluss (P1) der Prüfspannungsquelle verbunden ist, der im normalen Betriebszustand auf dem Bezugspotential (Masse) und zur Prüfung auf einem positiven Potential (H) nahe der Betriebsspannung (Vcc) der Schaltung liegt, und wobei an dem anderen Anschluss des Widerstandsspannungsteilers (R3, R4) die Betriebsspannung (Vcc) anliegt, und
der Ausgang des Differenzverstärkers (DV) mit dem Eingang (P2) eines A/D-Wandlers des Mikroprozessors (µP) verbunden ist, der einen zwischen dem Bezugspotential (L) und dem positiven Potential (H) der Prüfspannungsquelle umschaltbaren Ausgang (P1) hat, sowie einen Speicher für ein den Ablauf und die Auswertung der Funktionsprüfung steuerndes Programm umfasst.

## Claims

1. Method for testing the function of an electrochemical, electrolyte-containing gas sensor (S), in particular a carbon monoxide sensor, the electrodes (C, W) of which are connected to a differential amplifier (DV), the output signal of which is a measure for a gas concentration, wherein, in order to test the function, a test voltage which, on the basis of the normal operating state of the sensor, is inverted and greater than the electrolysis voltage of the electrolyte, is applied to the electrodes and the output signal of the differential amplifier is measured, at least after the test voltage is switched off, wherein, after the test voltage is switched off, the output signal is measured at a predefined point in time (T7) while the current supplied by the sensor (S) is decaying, the measured value (MW4) is compared with a predefined minimum value (B1) and, if this minimum value is not met, an error signal is generated.

2. Method according to claim 1, wherein the output signal is measured at at least one predefined point in time (T3, T5) after the test voltage is applied and before the sensor discharges, and the additional measured value (MW2, MW3) is compared with a predefined upper reference value (B2) and wherein, if this upper reference value (B2) is not met, an additional error signal is generated.

3. Method according to either claim 1 or claim 2, wherein the output signal is measured before the test voltage is applied and this third measured value (MW1) is compared with the predefined minimum value (B1) and wherein, if this minimum value (B1) is exceeded, a third error signal is generated.

4. Sensor system, designed to carry out a method for testing the function of an electrochemical, electrolyte-containing gas sensor (S), according to any of claims 1 to 3, wherein the sensor system comprises:
an electrochemical, electrolyte-containing gas sensor (S), in particular a carbon monoxide sensor;
a differential amplifier (DV);
a clamping diode (D); and
a microprocessor (µP) comprising a comparator; wherein
electrodes (C, W) of the gas sensor (S) are connected to the inputs of the differential amplifier (DV) and can also be connected to a test voltage source in order to test the function, which test voltage source supplies a test voltage (H) which, on the basis of the normal operating state of the sensor (S), is inverted and greater than the electrolysis voltage of the electrolyte;
a first electrode (W) is connected to the reference potential (earth) by means of the clamping diode (D),
in the normal operating state, a second electrode (C) is at a potential of between 0.1 V and 1 V, which is positive with respect to the reference potential, and can be switched to a positive potential of the test voltage source, which potential is greater compared therewith, for testing the function, since the second electrode (C) of the gas sensor (S) is connected to the tap (X) of a resistive voltage divider (R3, R4), one terminal of which is connected to a terminal (P1) of the test voltage source, which terminal, in the normal operating state, is at the reference potential (earth) and, in order to test for positive potential (H), is close to the operating voltage (Vcc) of the circuit, and wherein the operating voltage (Vcc) is applied to the other terminal of the resistive voltage divider (R3, R4), and
the output of the differential amplifier (DV) is connected to the input (P2) of an A-D converter of the microprocessor (µP) which has an output (P1) which can be switched between the reference potential (L) and the positive potential (H) of the test voltage source, and a memory for a program that controls the execution and evaluation of the testing of the function.

## Revendications

1. Procédé pour l'essai fonctionnel d'un capteur de gaz (S) électrochimique contenant un électrolyte, en particulier d'un capteur de monoxyde de carbone, dont les électrodes (C, W) sont reliées à un amplificateur différentiel (DV), dont le signal de sortie est une mesure d'une concentration de gaz, dans lequel, pour l'essai fonctionnel, une tension d'essai, qui est inversée par rapport à l'état de fonctionnement normal du capteur et est supérieure à la tension d'électrolyse de l'électrolyte, est appliquée sur les électrodes et, au moins après la coupure de la tension d'essai, le signal de sortie de l'amplificateur différentiel est mesuré, dans lequel après la coupure de la tension d'essai, le signal de sortie est mesuré à un moment (T7) prédéfini pendant la décroissance du courant fourni par le capteur (S), la valeur de mesure (MW4) est comparée à une valeur moyenne (B1) prédéfinie et, lorsque cette valeur minimale n'est pas atteinte, un signal d'erreur est généré.

2. Procédé selon la revendication 1, dans lequel le signal de sortie est mesuré à au moins un moment (T3, T5) prédéfini après l'application de la tension d'essai et avant la décharge du capteur et l'autre valeur de mesure (MW2, MW3) est comparée à une valeur de référence supérieure (B2) prédéfinie et dans lequel, lorsque cette valeur de référence supérieure (B2) n'est pas atteinte, un autre signal d'erreur est généré.

3. Procédé selon la revendication 1 ou 2, dans lequel le signal de sortie est mesuré avant l'application de la tension d'essai et cette troisième valeur de mesure (MW1) est comparée à la valeur minimale (B1) prédéfinie et dans lequel, lorsque cette valeur minimale (B1) est dépassée, un troisième signal d'erreur est généré.

4. Système de capteur, conçu pour mettre en oeuvre un procédé pour l'essai fonctionnel d'un capteur de gaz (S) électrochimique contenant un électrolyte selon l'une quelconque des revendications 1 à 3, dans lequel le système de capteur comporte :
un capteur de gaz (S) électrochimique contenant un électrolyte, en particulier un capteur de monoxyde de carbone ;
un amplificateur différentiel (DV) ;
une diode de blocage (D) ; et
un microprocesseur (µP) comprenant un comparateur ; dans lequel
les électrodes (C, W) du capteur de gaz (S) sont reliées d'une part aux entrées de l'amplificateur différentiel (DV) et peuvent être reliées d'autre part, pour l'essai fonctionnel, à une source de tension d'essai, qui fournit une tension d'essai (H) qui est inversée par rapport à l'état de fonctionnement normal du capteur (S) et supérieure à la tension d'électrolyse de l'électrolyte ;
une première électrode (W) est reliée au potentiel de référence (masse) par l'intermédiaire de la diode de blocage (D),
une deuxième électrode (C) dans l'état de fonctionnement normal se situe sur un potentiel, positif par rapport au potentiel de référence, compris entre 0,1 V et 1 V et, pour l'essai fonctionnel, peut être commutée sur un potentiel positif de la source de tension d'essai supérieur à celui-ci, étant donné que la deuxième électrode (C) du capteur de gaz (S) est reliée à la prise (X) d'un diviseur de tension de résistance (R3, R4), dont une connexion est reliée à une connexion (P1) de la source de tension d'essai, laquelle connexion, dans l'état de fonctionnement normal, se situe sur le potentiel de référence (masse) et, pour l'essai, sur un potentiel positif (H) à proximité de la tension de service (Vcc) du circuit, et dans lequel la tension de service (Vcc) s'applique sur l'autre connexion du diviseur de tension de résistance (R3, R4), et
la sortie de l'amplificateur différentiel (DV) est reliée à l'entrée (P2) d'un convertisseur analogique/numérique du microprocesseur (µP), qui présente une sortie (P1) pouvant commuter entre le potentiel de référence (L) et le potentiel positif (H) de la source de tension d'essai, ainsi qu'une mémoire pour un programme commandant le déroulement et l'évaluation de l'essai fonctionnel.
